# EUROPÄISCHE PATENTSCHRIFT

(11) **EP 2 029 089 B1**
(45) Veröffentlichungstag und Bekanntmachung des Hinweises auf die Patenterteilung: **21.07.2010**
(21) Anmeldenummer: 07729463.5
(22) Anmeldetag: 24.05.2007
(51) Int. Cl.: A61K 8/06, A61K 8/34, A61K 8/37, A61Q 19/00, A61K 31/047, A61K 31/215

(54) **O/W EMULSION ZUR PFLEGE DER HAND**
O/W EMULSION FOR CARING FOR HANDS
ÉMULSION H/E POUR LE SOIN DE LA MAIN

(30) Priorität: 08.06.2006 DE 102006027138
(43) Veröffentlichungstag der Anmeldung: 04.03.2009
(73) Patentinhaber: Beiersdorf AG, 20253 Hamburg (DE)
(72) Erfinder: KRÖPKE, Rainer, 22869 Schenefeld (DE); HAHN, Ingo, 25421 Pinneberg (DE); LEMM, Christel, 21614 Buxtehude (DE)
(86) Internationale Anmeldenummer: PCT/EP2007/055032
(87) Internationale Veröffentlichungsnummer: WO 2007/141141

(56) Entgegenhaltungen:
- WO-A-02/19973
- WO-A-03/017952
- WO-A-03/045349
- WO-A-2005/004936
- KR-A- 20040 076 909
- US-A1- 2004 156 874

## Beschreibung

Die Erfindung beschreibt eine O/W-Emulsion umfassend Fettalkohol und/oder Fettalkoholgemische, mindestens ein Befeuchtungsmittel, einen Phosphatemulgator und mindestens ein Glycerinester einer Fettsäure. Der Anteil an Befeuchtungsmittel, bevorzugt Glycerin, beträgt mindestens 25 Gew.%, bezogen auf die Gesamtmasse der Emulsion.
Die Emulsion ist zur Herstellung eines Mittels zur sofortigen Pflege, Verschönerung und/oder Behandlung spröder, rissiger und/oder entzündeter Haut, bevorzugt der Hand, besonders geeignet. Bevorzugte Ausführungsformen der erfindungsgemässen Emulsion zeigen eine "Sofort-Wirkung" gegen sehr trockenen Haut bzw. spröde/rissige Haut.

Es sind zahlreiche Handcremes, Lotionen und Gele, die auf der menschlichen Haut und bevorzugt der Hand aufgetragen werden können, bekannt.
Beispielsweise Lindesa O Handcreme, Logona - Daily Care Handcreme oder pH5 Eucerin Handcreme. Die Handcremes eignen sich zur Pflege nach der Arbeit bei trockener und entfetteter Haut, zur intensive Pflege für rauhe und strapazierte Hände. Die Pakungsauslobungen der im Markt erhältlichen Handcremes sind vielfältig, wie z.B. "Schenkt Ihren Händen Schutz, Pflege und Feuchtigkeit auch unter schwierigen Bedingungen wie Kälte oder anderen widrigen Umwelteinflüssen".

Die bekannten emulsionsbasierenden Handcremes enthalten neben Wasser oftmals Glycerin, Cetearyl Alcohol und Glycerylstearate.

Wünschenswert wäre es demnach eine Emulsion bereit zu stellen, die auf die Haut, bevorzugt der Hand, aufzutragen ist und die einen sofortigen Pflege- und/oder Verschönerungseffekt bei spröder, rissiger oder trockener Haut hervorruft.

WO 03045349 A2 offenbart eine O/W Emulsionen umfassend Fettalkohol (Cetylakohol), ein Befeuchtungsmittel (15 Gew.% Glycerin) und ein Glycerinester einer Fettsäure (Glycerylstearat).

KR 1020040076909 offenbart eine Massagecreme umfassend Fettalkohol (Stearylalkohol), Befeuchtungsmittel (max. 20% Glycerin und Propylenglycol) und ein Glycerinester einer Fettsäure (Glycerylstearat).

US 20040156874 A1 offenbart eine kosmetische Zubereitung umfassend Fettalkohole, Befeuchtungsmittel (40% Harnstoff, 1-5%Propylenglycol) und ein Glycerinester einer Fettsäure (Glycerylstearat).

WO 03017952 A1 offenbart O/W Emulsionen umfassend Fettalkohol (Cetylstearylalkohol), ein Befeuchtungsmittel (7 Gew.% Glycerin) und ein Glycerinester einer Fettsäure (Glycerylstearatcitrat).

Es ist darüber hinaus wünschenswert neben den bekannten Pflegeprodukten eine weitere kosmetische Zubereitung auf Emulsionsbasis zur Verfügung zu stellen, die die Auswahl der auf dem Markt erhältlichen Zubereitungen bereichert.

Die Erfindung umfasst eine O/W-Emulsionen umfassend Fettalkohol und/oder Fettalkoholgemische, mindestens ein Glycerinester einer Fettsäure, einen Phosphatemulgator und mindestens ein Befeuchtungsmittel.
Erfindungsgemäß liegt das Befeuchtungsmittel, idealerweise Glycerin, in hoher Konzentration vor, über 25 Gew.%. Dies führt in und auf der Haut zu einer sehr guten und nachhaltige Befeuchtung. In dieser hohen Konzentration hat das Befeuchtungsmittel, bevorzugt Glycerin, auch weichmachende Eigenschaften auf die damit eingecremte Haut.
Andere Pflegestoffe, die der Emulsion bevorzugt zugesetzt sein können, können dadurch in die rissige bzw. spröde Haut leichter und besser eindringen und somit den Pflegeeffekt nachhaltig unterstützen.

Nachteilig war es bisher, dass ein hoher Anteil an Befeuchtungsmitteln in Emulsionen dazu führte, dass die Pflegeemulsionen eine unakzeptable Konsistenz und eine vom Anwender nicht gewollte Klebrigkeit aufwiesen.
Erfindungsgemäß wurde überraschenderweise dieser Nachteil überwunden. Durch die Kombination von mindestens einem Glycerinester, insbesondere Glyceryllaurat, Fettalkohol bzw. Fettalkoholgemischen und einem hohen Anteil an Befeuchtungsmitteln von mehr als 15 Gew.%, insbesondere mehr als 20 Gew.%, wurde eine Verbesserung der Konsistenz und vor allem der Klebrigkeit der Emulsion erreicht.
Es ist die Verwendung einer Kombination von mindestens einem Fettsäureglycerinester, insbesondere Glyceryllaurat, mindestens einem Fettalkohol und mehr als 15 Gew.% an Befeuchtungsmitteln, insbesondere Glycerin, zur Verbesserung der Konsistenz und/oder der Klebrigkeit von kosmetischen Zubereitungen damit bevorzugt gegeben.

Hautfeuchtigkeit ist ein Begriff aus der Kosmetikindustrie. Gesunde Haut besitzt eine natürliche Feuchtigkeit. Erst wenn die menschliche Haut Anomalien in Bezug auf Trockenheit aufweist, kommt mangelnde Hautfeuchtigkeit zum Tragen. Außer krankhaft bedingten Ursachen spielt auch das Alter der menschlichen Haut sowie die Pigmentierung eine Rolle. Ein wichtige Rolle bei der Feuchtigkeit der Haut spielen Feuchthaltefaktoren wie z.B. Urea. Diese können der Haut durch Hautpflegemittel zugeführt werden.

Normalerweise benötigt die menschliche Haut keinerlei Hilfsmittel zur Erhaltung der natürlichen Feuchtigkeit. Jedoch tragen ungesunde Lebensweise, trockene Luft (besonders in Solarien und geheizten Innenräumen), Umwelteinflüsse, Stress und lange Sonnenbäder zum Entzug von Feuchtigkeit bei. Auch lange und heiße Wannenbäder und Waschmittelreste in der Kleidung verursachen den Verlust der wichtigen Bestandteile des Hydrolipidsystems der Haut. Um einer Austrocknung der Haut vorzubeugen verwendet man Feuchtigkeitscremes. Auch rückfettende Seifen und weitgehend seifenfreie Waschmittel geben das Fett wieder an die Haut zurück. So wird sie wieder glatter und geschmeidiger.Hier werden insbesondere Körperreinigungsmittel empfohlen, die den natürlichen Säureschutzmantel der Haut nicht zerstören (pH der Haut = 5,5).

In manchen Wohnungen, deren Belüftung durch negative Umweltbedingungen nicht wie üblich möglich ist, können zur Unterstützung einer gesunden Hautfeuchtigkeit auch sogenannte Luftbefeuchter eingesetzt werden.

Durch die Bereitstellung der erfindungsgemäßen Emulsion hat der Anwender nun eine weitere Möglichkeit zur Hand, insbesondere für die Hand, seine trockene, spröde oder rissige Haut zu pflegen und ihr Feuchtigkeit zu zuführen.

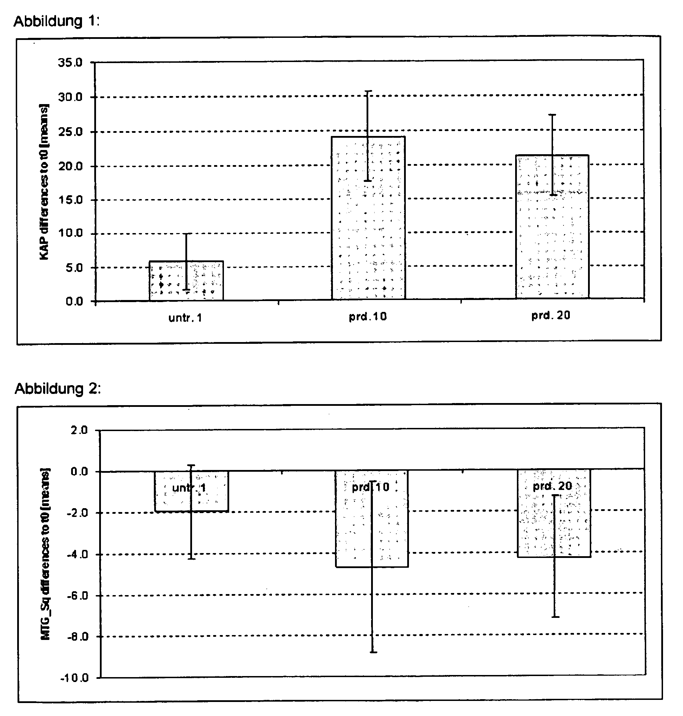

Wirksamkeitstests, wie in den Abbildung 1 und 2 dargelegt, belegen die Hautbefeuchtungsleistung (KAP) der erfindungsgemäßen Emulsion (prod 10) gegenüber unbehandelter Haut (untr.1) und gegenüber Produkten des Standes der Technik (prod 20, Neutrogena® Handcreme Konzentrat). Die erfindungsgemäße Emulsion, mit bis zu 30 Gew.% Glycerin, steigert die Hautfeuchte um 417% (Abbildung 1).

Ebenso wurden Werte für die Hautglättung (MTG) ermittelt, die belegen, dass die erfindungsgemäße Emulsion wirksam für die kosmetische Pflege und Verschönerung der Haut eingesetzt werden kann, insbesondere gegen trockene, spröde oder rissige Haut.
Die Werte zeigen, dass die erfindungsgemäße Emulsion die Hautglätte nach 7 Tagen um 235% verbessert (Abbildung 2).

Die durchgeführten Studien zeigen eine Effektivitätsteigerung in der Hautpflege aufgrund der verbesserten Hautfeuchte als auch in einer verringerten Rauhigkeit der Haut. Die erfindungsgemäßen Emulsionen können daher zu einer Verbesserung bzw. Steigerung der Hautfeuchte (KAP) und/oder Hautglättung (MTG) verwendet werden.

Eine Steigerung der Hautfeuchte (KAP) um bis zu 417% und/oder eine Steigerung der Hautglättung (MTG) um bis zu 235% ist mit Hilfe der erfindungsgemäß bevorzugten Emulsion möglich.

Die Studien zu a. Hautfeuchte(Skin Moisture (parameter code: KAP)) und b. Hautglätte ((Skin Roughness (parameter code: MTG)) wurden wie folgt durchgeführt:
a. Hautfeuchte(Skin Moisture (parameter code: KAP))
   Messgerät: Corneometer CM 825 (Courage + Khazaka, Cologne, Germany)
   Parameter: permittivity [a.u.] = arbitrary units, median resulting from repeated measurement Wiederholung (Repetitions): 10 Messungen per Area
   Richtlinie, Vorgabe: "EEMCO Guidance for the Assessment of Stratum Corneum Hydration: Electrical Methods" by E. Berardesca (published in Skin Research and Technology, 1997, 3: 126-132)
b. Hautglätte (Skin Roughness (parameter code: MTG))
   Messgerät: µCAD supplied by GFMesstechnik (Teltow, Germany)
   Prinzip: Optical Topometry on silicone imprints (negative impressions of the topographic features of the skin made with Silflo^{®})
   Parameter: root mean square deviation of the surface Sq [µm], original values Wiederholung: 1 imprint per area
   Richtlinie, Vorgabe: "EEMCO* guidance for the assessment of skin topography" from J.L. Lévêque (published in J Eur Acad Dermatol Venerol, 1999, 12: 103-114

Die Emulsionen lassen sich zu einer Verbesserung bzw. Steigerung der Hautfeuchte (KAP) und/oder Hautglättung (MTG) verwenden.
Es ist somit bevorzugt die erfindungsgemäße Emulsion zur Herstellung eines Mittels zur Pflege oder Linderung spröder, rissiger und/oder entzündeter Haut, bevorzugt der Hand, zu verwenden.

Als Befeuchtungsmittel, auch als Moisturizer bezeichnet, werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Lanolin und deren Ester, Lanolinalkohol, Sorbitol, Milchsäure und deren Salze, Pyrrolidoncarbonsäure und deren Salze und Harnstoff. Weitere Befeuchtungsmittel sind beispielsweise polymere Moisturizer aus der Gruppe der wasserlöslichen und/oder in Wasser quellbaren und/oder mit Hilfe von Wasser gelierbaren Polysaccharide. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.
Bevorzugtes Befeuchtungsmittel ist Glycerin. Der Anteil an Glycerin in der Emulsion beträgt mindestens 25 Gew.%, bezogen auf die Gesamtmasse der Emulsion.
Glycerin hat sich als ein sehr effektives Befeuchtungsmittel gezeigt, weil es u.a. bei diesen hohen Anteilen einen messbaren Sofort-Effekt hinsichtlich Hautbefeuchtung und Hautglättung zur Folge hat.

Der Anteil an Fettalkohol bzw. Fettalkoholgemischen beträgt bevorzugt mindestens etwa 2 Gew.%, bezogen auf die Gesamtmasse der Emulsion.
Bevorzugt ist der Anteil an Fettalkohol bzw. Fettalkoholgemischen maximal etwa 10 Gew.%, bezogen auf die Gesamtmasse der Emulsion. Als insbesondere vorteilhaft hat sich gezeigt, wenn die Fettalkohole bzw. Fettalkoholgemische einen Schmelzpunkt im Bereich von etwa 32°C bis 56°C aufweisen.

Durch die Kombination mit Fettalkoholen bzw. Fettalkoholgemischen, insbesondere mit einem Schmelzpunkt von etwa 32-56 °C, wird eine sehr gute physikalische Emulsionsstabilität und ein "schmelzendes" Verhalten der Textur auf der Haut erreicht, was wiederum die ansonsten immense Klebrigkeit einer Emulsion mit diesen hohen Glycerinkonzentrationen entscheidend abmildert.

In einem weiteren Aspekt der Erfindung wird der Anteil an Fettsäureglycerinester bevorzugt mindestens etwa 0,1 Gew.%, bevorzugt mindestens etwa 0,5 Gew.%, bezogen auf die Gesamtmasse der Emulsion, gewählt.
Bevorzugt ist der Anteil an Fettsäureglycerinester bevorzugt maximal etwa 5 Gew.%, bevorzugt maximal etwa 3 Gew.%, bezogen auf die Gesamtmasse der Emulsion.

Als insbesondere vorteilhaft sind die Fettsäureglycerinester mit einer C-Kettenlänge von weniger als 22 auszuwählen. Bevorzugter Fettsäureglycerinester ist dabei Glyceryllaurat. Die Kombination aus Fettalkohol bzw. Fettalkoholgemischen, insbesondere mit einem Schmelzpunkt von etwa 32-56 °C, Fettsäureglycerinester, insbesondere Glyceryllaurat, und mehr als 15 Gew.% an Befeuchtungsmitteln, insbesondere Glycerin, in einer O/W-Emulsion führt zu einer Verbesserung der Hautbefeuchtung und Hautglättung (s. Test, Abb. 1 und 2) und gleichzeitig zu einer Verringerung der Klebrigkeit und damit zu einer angenehmeren Applikation und Handhabung.

In einem weiteren bevorzugten Aspekt umfasst die erfindungsgemäße O/W-Emulsion
- mindestens etwa 2 Gew.%, bevorzugt maximal etwa 10 Gew.%, Fettalkohol und/oder Fettalkoholgemische mit einem Schmelzpunkt im Bereich von etwa 32°C bis 56°C,
- mindestens etwa 0,1 Gew.%, bevorzugt mindestens etwa 0,5 Gew.%, bevorzugt maximal 5 Gew.%, Fettsäureglycerinester mit einer C-Kettenlänge von weniger als 22, bevorzugt Glyceryllaurat und
- mindestens 25 Gew.%, eines Befeuchtungsmittels, bevorzugt Glycerin.

Die Anteile beziehen sich jeweils auf die Gesamtmasse der Emulsion.

Der Emulsion ist mindestens ein Phosphatemulgator zugesetzt. Bevorzugt ist der Anteil an Phosphatemulgatoren im Bereich von mindestens etwa 0,1, bevorzugt mindestens 0,5 Gew.%, bezogen auf die Gesamtmasse der Emulsion, zu wählen.

Der Anteil an Phosphatemulgatoren liegt bevorzugt bei maximal etwa 5 Gew.%, bevorzugt maximal etwa 2,5 Gew.%, bezogen auf die Gesamtmasse der Emulsion.

Als Phosphatemulgatoren werden bevorzugt Trilaureth-4 Phosphat und/oder Triceteareth-4 Phosphat gewählt.

Die Kombination der erfindungsgemäßen Bestandteile mit mindestens einem Phosphatemulgator zeigt eine stabilisierende Wirkung auf O/W-Emulsionen mit sehr hohen Befeuchtungsmitteln. Darüber hinaus sorgen die Phosphatemulgatoren für ein weiches, geschmeidiges Hautgefühl und verbessern die Verteilbarkeit der Textur.

Dieser Vorgang ist vergleichbar mit dem Lecithin, chemische Bezeichnung: Phosphatidylcholin, ein Phosphoglyzerid, welches in der Lebensmittelindustrie als Emulgator zur Stabilisierung von Fett-in-Wasser-Gemischen verwendet wird.

In einem weiteren bevorzugten Aspekt umfasst die erfindungsgemäße O/W-Emulsion
- mindestens etwa 2 Gew.%, bevorzugt maximal 10 Gew.%, Fettalkohol und/oder Fettalkoholgemische mit einem Schmelzpunkt im Bereich von etwa 32°C bis 56°C,
- mindestens etwa 0,1 Gew.%, bevorzugt mindestens etwa 0,5 Gew.%, bevorzugt maximal bis etwa 5 Gew.%, Fettsäureglycerinester mit einer C-Kettenlänge von weniger als 22, bevorzugt ist Glyceryllaurat, - mindestens etwa 0,1 Gew.%, bevorzugt mindestens etwa 0,5 Gew.%, bevorzugt maximal bis 5 Gew.%, Phosphatemulgatoren, bevorzugt Trilaureth-4 Phosphat und/oder Triceteareth-4 Phosphat, und
- mindestens 25 Gew.%, eines Befeuchtungsmittels, bevorzugt Glycerin.

Die Anteile beziehen sich jeweils auf die Gesamtmasse der Emulsion.

In einem bevorzugten Aspekt können der erfindungsgemäßen O/W-Emulsion weitere hautpflegende, wundheilende, entzündungshemmende und/oder schmerzlindernde Stoffe zugesetzt werden.
Bevorzugt werden Panthenol und/oder Calendulaöl gewählt.

Eine Emulsion der Panthenol zu einem Anteil von etwa 0,1 bis 5 Gew.%, bevorzugt etwa 0,5 bis 2 Gew.%, und Calendulaöl im Bereich von etwa 0,01 bis 5 Gew.%, bevorzugt 0,1 bis 4 Gew.%, bezogen auf die Gesamtmasse der Emulsion, zugesetzt sind, ist besonders bevorzugt.

In einem weiteren bevorzugten Aspekt umfasst die erfindungsgemäße O/W-Emulsion daher
- 2 bis 10 Gew.% Fettalkohol und/oder Fettalkoholgemische mit einem Schmelzpunkt im Bereich von etwa 32°C bis 56°C,
- 0,1 bis 5 Gew.%, bevorzugt 0,5 bis 3 Gew.%, Fettsäureglycerinester mit einer C-Kettenlänge von weniger als 22, bevorzugt Glyceryllaurat,
- mindestens 25 Gew.%, eines Befeuchtungsmittels, bevorzugt Glycerin,
- Phosphatemulgatoren, bevorzugt Trilaureth-4 Phosphat und/oder Triceteareth-4 Phosphat,
- 0,1 bis 5 Gew.%, bevorzugt 0,5 bis 2 Gew.%, Panthenol und
- etwa 0,01 bis 5 Gew.%, bevorzugt 0,1 bis 4 Gew.%, Calendulaöl.

Panthenol, auch unter den Namen Dexpanthenol, Pantothenol, D-Pantothenylalkohol oder Provitamin B5 bekannt, wird durch die chemische Bezeichnungen (R)-2,4-Dihydroxy-N-(3-hydroxypropyl)-3,3-dimethylbutansäureamid, (R)-2,4-Dihydroxy-3,3-dimethyl-buttersäure-3-hydroxypropylamid beschrieben.

Das Panthenol ist die alkoholische und in Kosmetika stabilere Form des B-Vitamins Pantothensäure. Panthenol wird in der Haut in Pantothensäure umgewandelt. In Hautpflegepräparaten soll es für eine verbesserte Hautfeuchtigkeit sorgen, indem es tief in die unteren Hautschichten eindringt und dort Wasser bindet. Diese Wirkung entfaltet das Panthenol zusätzlich zu seiner normalen Funktion im Organismus.

Panthenol (Dexpanthenol) fördert den Energiestoffwechsel der Hautzellen und deren optimale Ernährung. Es regt, ähnlich wie das Vitamin A, die Teilung der Hautzellen an. Da es die Haut beruhigt und heilend wirkt, hilft es auch bei einem akutem Sonnenbrand. Für die Haare hat Panthenol zusätzlich noch eine ganz besondere Bedeutung.

Calendulaöl, botanischer Name Calendula officinalis L., entstammt der Pflanzenfamilie Asteraceae - Korbblütler. Hinter der Bezeichnung der Calendula versteckt sich die Ringelblume. Seit vielen Jahrhunderten werden aus ihr Salben, Tee und andere Heilmittel hergestellt. Der lateinische Name "calendula" stammt vom lateinischen Wort "calendae" ab, dem ersten Tag im Monat und da die Ringelblume an ganz vielen "calendis" also Monaten blüht, wurde sie Calendula genannt.

Das Calendulaöl ist ein Mazerat, das meist in Oliven- oder Sojaöl ausgezogen wird. Mazerate sind Pflanzenöle, in denen eine Heilpflanze in einem Öl einige Zeit der Sonne ausgesetzt und ihre Wirkstoffe sich in das Pflanzenöl übertragen haben. Sojaöl ist als Bestandteil von Pflegeemulsionen daher häufig nur der "Träger" für Calendulaöl.

Ringelblumenöl, Calendulaöl, wirkt wundheilend, entzündungshemmend und schmerzlindernd. Es ist besonders hilfreich bei trockener und rissiger Haut, pflegt aber auch die zarte und empfindliche Haut. Bei rheumatischen Beschwerden und schlecht heilenden Wunden unterstützt es den Heilungsverlauf.

Aufgrund der geschilderten Vorteile und Inhaltststoffe stellen die erfindungsgemäßen Emulsionen vorteilhaft die Basis einer kosmetischen Zubereitung dar. Erfindungsgemäß umfasst der Begriff Emulsion auch kosmetische Zubereitungen auf Basis einer entsprechenden Emulsion.
Die Emulsion oder die Zubereitung diese enthaltend kann wiederum vorteilhaft in einer für die topische Applikation geeigneten Form vorliegen. Beispielsweise kann die Zusammensetzung in Form einer Creme, einer Lotion, eines Gels, einer Salbe, einer Tinktur, einer Milch, eines Balsams, eines mit der Zusammensetzung imprägnierten Pflasters, eines mit der Zusammensetzung imprägnierten Tuchs, einer mit der Zusammensetzung imprägnierten Textilie, eines mit der Zusammensetzung imprägnierten Pads, eines Sprays, eines Aerosols, eines Roll-on, eines Stifts, einer Soft Solid, eines Puders oder eines Pudersprays vorliegen.

Bevorzugt ist die Emulsion zum Auftragen auf die menschliche Haut, bevorzugt die Hand, zu verwenden. Sie wird daher bevorzugt als Handcreme angeboten und verwendet.

Die erfindungsgemäße Emulsion lässt sich vorteilhaft zur Pflege und/oder Verschönerung trockener, spröder, rissiger und/oder entzündeter Haut, bevorzugt der Hand, verwenden.

Dabei ist erstaunlicherweise festgestellt worden, dass die erfindungsgemäße Emulsion die zu pflegende Haut sofort beruhigt und sehr schnell und nachhaltig den gestörten Feuchtigkeitshaushalt der Haut wieder herstellt.
Als "Sofort-Effekt" wird erfindungsgemäß eine Verbesserung der Hautfeuchte oder Hautglätte innerhalb einer Stunde bis zu 7 Tagen bezeichnet. Dies insbesondere nach einmaliger bzw. regelmäßiger Applikation der erfindungsgemäßen Emulsion in ausreichender Menge auf die zu pflegende Haut.
Eine ausreichende Menge ist die üblicherweise auf die entsprechenden Hautpartien aufgetragene Menge. Dies kann bei einer Handcreme in etwa Mengen von 0,5 bis 1 g bei Köperlotionen durchaus das 10 bis 100 fache sein, wobei hier auch die Größe der zu pflegenden Hautareale eine Rolle spielt.
Ein Verfahren zur Verbesserung der Hautfeuchtigkeit und/oder Hautglätte ist damit bevorzugt. Es wird dazu die erfindungsgemäße Emulsion oder eine kosmetische Zubereitung diese enthaltend in ausreichender Menge auf die Haut aufgetragen. Nach einmaliger als auch bevorzugt nach Applikation einmal täglich zeigt sich ein überraschender "Sofort-Effekt" innerhalb einer Stunde sowie ein nachhaltiger Effekt auch noch nach einer Woche. Verbesserung bedeutet erfindungsgemäß eine Zunahme oder positive Änderung der Hautfeuchte oder Hautglätte gegenüber dem Zustand der Haut ohne die Auftragung der erfindungsgemäßen Emulsion.
Insbesondere zeigt sich dieser "Sofort-Effekt" bei den kosmetischen Zubereitungen umfassend die erfindungsgemäße O/W-Emulsion mit
- 2 bis 10 Gew.% Fettalkohol und/oder Fettalkoholgemische mit einem Schmelzpunkt im Bereich von etwa 32°C bis 56°C,
- 0,1 bis 5 Gew.%, bevorzugt 0,5 bis 3 Gew.%, Fettsäureglycerinester mit einer C-Kettenlänge von weniger als 22, bevorzugt Glyceryllaurat,
- mindestens 25 Gew.%, eines Befeuchtungsmittels, bevorzugt Glycerin,
- Phosphatemulgatoren, bevorzugt Trilaureth-4 Phosphat und/oder Triceteareth-4 Phosphat,
- 0,1 bis 5 Gew.%, bevorzugt 0,5 bis 2 Gew.%, Panthenol und
etwa 0,01 bis 5 Gew.%, bevorzugt 0,1 bis 4 Gew.%, Calendulaöl.

Die Emulsion ist als Kosmetikum zu verwenden und kann darüber hinaus zur Herstellung eines Mittels zur, bevorzugt sofortigen, Pflege, Verschönerung und/oder Behandlung spröder, rissiger und/oder entzündeter Haut, bevorzugt der Hand, verwendet werden. Trockene und empfindliche Haut bleibt so länger gesund, schön und geschmeidig.

Die erfindungsgemäße Mittel sind dabei keine therapeutisch wirksamen Mittel.

Die Wasserphase der erfindungsgemäßen Emulsion oder Zubereitungen, diese umfassend, kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Ethylenglykol, Ethylenglykolmonoethyl- oder -monobutylether, Propylenglykolmonomethyl, monoethyl- oder -monobutylether, Diethylenglykolmonomethyl- oder -monoethylether und analoge Produkte, Polymere, Schaumstabilisatoren sowie Elektrolyte, wobei diese nicht als oben bezeichnete Befeuchtungsmittel gelten.

Die erfindungsgemäße Emulsion oder erfindungsgemäß verwendete kosmetische Zubereitungen können selbstverständlich kosmetische Hilfsstoffe enthalten, wie sie üblicherweise in solchen Zubereitungen verwendet werden, z.B. Konservierungsmittel, Bakterizide, UV-Filter, Antioxidantien, wasserlösliche Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

In den nachfolgenden Beispielen sind verschiedenen erfindungsgemäße Emulsionen bzw. Zubereitungen diese enthaltend und deren Verwendung dargestellt. Die angegebenen Anteilswerte sind Gewichtsprozente bezogen auf die Gesamtmasse der Zubereitung, sofern nichts anderes angegeben ist:

### Beispiele

### O/W-Emulsion

| | 3 | 4 | 5 |
|---|---|---|---|
| lyceryllaurat | 1,0 | 0,5 | 0,75 |
| Ceteareth-3 | 2,0 | 2,5 | 0,5 |
| Triceteareth-4 Phosphat | 1,2 | 1,7 | 2,5 |
| Petrolatum (Vaseline) | 4,0 | 5,0 | 3,2 |
| Calendulaöl | 0,1 | 3,5 | 1,75 |
| Dimethicon | 3,5 | 2,0 | 1,0 |
| Myristylalkohol | 4,0 | 1,0 | 0,75 |
| Sorbitanstearat | 2,5 | 3,5 | 1,25 |
| Cetylalkohol | 4,0 | 1,0 | 2,75 |
| Panthenol | 0,5 | 2,0 | 0,25 |
| Isopropylpalmitat | 2,5 | 3,0 | 7,5 |
| Parfum | q,s, | q,s, | q,s, |
| Methylparaben | 0,30 | 0,3 | 0,4 |
| Propylparaben | 0,15 | 0,15 | 0,1 |
| Phenoxyethanol | 0,7 | 0,5 | 0,1 |
| Glycerin | 26,5 | 36,5 | 46,5 |
| Natriumcarbomer | 0,3 | 0,15 | 0,65 |
| Wasser | ad 100 | ad 100 | ad 100 |

Bevorzugt verwendbar als Handcreme, Fußcreme, Mousseapplikation mit/ohne Treibgas oder Pflegelotion.

### O/W-Emulsion

| | 8 | 9 | 10 |
|---|---|---|---|
| Polyethylenglycol(21)stearylether | 2,5 | 2 | 1,5 |
| Polyethylenglycol(2)stearylether | 5,5 | 3 | 7,5 |
| Cetearylglucosid | --- | --- | --- |
| Glycerylstearat | 3,0 | 2,5 | 5,0 |
| Dimethicon | 5,0 | 2,0 | 5,0 |
| Behenylalkohol | 1,0 | 1,0 | --- |
| Stearylalkohol | 1,0 | 2,0 | 1,75 |
| Cetylstearylalkohol | 1,0 | --- | 2,0 |
| Myristylalkohol | 1,0 | 2,0 | 0,25 |
| Calendulaöl | 1,5 | 2,0 | 0,75 |
| Trilaureth-4 Phosphat | 0,75 | 3,0 | 0,25 |
| Glycerin | 35 | 26 | 55 |
| Panthenol | 0,75 | 0,25 | 0,1 |
| Parfum | q,s, | q,s, | q,s, |
| Methylparaben | 0,05 | 0,3 | 0,4 |
| Propylparaben | 0,25 | 0,15 | --- |
| Sorbitol | 1,0 | --- | 5,0 |
| Urea | --- | 3,0 | -- |
| Wasser | ad 100 | ad 100 | ad 100 |

### O/W-Emulsion

| | 15 |
|---|---|
| Glycerylsteratcitrat | 0,3 |
| Polyethylenglycol(20)cetearylether | -- |
| Triglycerinmethylglucosedistearat | 2,5 |
| Glyceryllaurat | 5,0 |
| Cyclomethicon | -- |
| Dimethicon | 0,2 |
| Behenylalkohol | 0,75 |
| Dicaprylylcarbonat | 5,0 |
| Stearylalkohol | 0,75 |
| Cetylstearylalkohol | 2,5 |
| Tocopherol | 0,1 |
| Octyldodecanol | 0,25 |
| Panthenol | 0,1 |
| Carbomer | --- |
| Parfum | q,s, |
| Caprylic/Capric Triglycerid | 10,0 |
| Methylparaben | 0,4 |
| Propylparaben | --- |
| Calendulaöl | 0,1 |
| lodopropynylbutylcarbamat | 0,1 |
| Phenoxyethanol | --- |
| Sorbitol | --- |
| Butylenglykol | 10,0 |
| Propylenglykol | --- |
| Glycerin | 35,0 |
| Wasser | ad 100 |

## Patentansprüche

1. O/W-Emulsion umfassend Fettalkohol und/oder Fettalkoholgemische, mindestens ein Befeuchtungsmittel, wobei der Anteil an Befeuchtungsmittel mindestens 25 Gew.% beträgt, bezogen auf die Gesamtmasse der Emulsion, mindestens ein Glycerinester einer Fettsäure und mindestens ein Phosphatemulgator.

2. Emulsion nach einem der vorstehenden Ansprüche, **dadurch gekennzeichnet, dass** als Befeuchtungsmittel Glycerin gewählt wird.

3. Emulsion nach einem der vorstehenden Ansprüche, **dadurch gekennzeichnet, dass** die Fettalkohole bzw. Fettalkoholgemische einen Schmelzpunkt im Bereich von etwa 32°C bis 56°C aufweisen.

4. Emulsion nach einem der vorstehenden Ansprüche, **dadurch gekennzeichnet, dass** der Anteil an Fettsäureglycerinester mindestens etwa 0,1 Gew.%, bevorzugt mindestens etwa 0,5 Gew.%, bevorzugt maximal etwa 5 Gew.%, insbesondere maximal 3 Gew.%, bezogen auf die Gesamtmasse der Emulsion, beträgt, und/oder der Anteil an Fettalkohol bzw. Fettalkoholgemischen mindestens etwa 2 Gew.%, bevorzugt maximal bis 10 Gew.%, bezogen auf die Gesamtmasse der Emulsion, gewählt wird.

5. Emulsion nach einem der vorstehenden Ansprüche, **dadurch gekennzeichnet, dass** der oder die Fettsäureglycerinester eine C-Kettenlänge von weniger als 22 aufweisen.

6. Emulsion nach einem der vorstehenden Ansprüche, **dadurch gekennzeichnet, dass** als Fettsäureglycerinester Glyceryllaurat gewählt wird.

7. Emulsion nach einem der vorstehenden Ansprüche, **dadurch gekennzeichnet, dass** der Anteil an Phosphatemulgatoren mindestens etwa 0,1 Gew.%, bevorzugt mindestens 0,5 Gew.%, bevorzugt maximal 5 Gew.%, insbesondere maximal 3 Gew.%, bezogen auf die Gesamtmasse der Emulsion, gewählt wird.

8. Emulsion nach einem der vorstehenden Ansprüche, **dadurch gekennzeichnet, dass** als Phosphatemulgatoren Trilaureth-4 Phosphat und/oder Triceteareth-4 Phosphat gewählt werden.

9. Emulsion nach einem der vorstehenden Ansprüche, **dadurch gekennzeichnet, dass** zusätzliche hautpflegende, wundheilende, entzündungshemmende und/oder schmerzlindernde Stoffe enthalten sind.

10. Emulsion nach Anspruch 9, **dadurch gekennzeichnet, dass** Panthenol, bevorzugt zu einem Anteil von 0,1 bis 5 Gew.%, insbesondere 0,5 bis 2 Gew.%, bezogen auf die Gesamtmasse der Emulsion, enthalten ist.

11. Emulsion nach einem der vorstehenden Ansprüche 9 bis 10, **dadurch gekennzeichnet, dass** Calendulaöl, bevorzugt etwa 0,01 bis 5 Gew.%, insbesondere 0,1 bis 4 Gew.%, bezogen auf die Gesamtmasse der Emulsion, enthalten ist.

12. Emulsion nach einem der vorstehenden Ansprüche, **dadurch gekennzeichnet, dass** Glycerin, Panthenol und Calendulaöl enthalten sind.

13. Emulsion nach einem der vorstehenden Ansprüche in Form einer Creme, einer Lotion, eines Gels, einer Salbe, einer Tinktur, einer Milch, eines Balsams, eines mit der Emulsion imprägnierten Pflasters, eines mit der Emulsion imprägnierten Tuchs, einer mit der Emulsion imprägnierten Textilie, eines mit der Emulsion imprägnierten Pads, eines Sprays, eines Aerosols, eines Roll-on, eines Stifts, einer Soft Solid, eines Puders oder eines Pudersprays.

14. Verwendung einer Emulsion nach einem der vorstehenden Ansprüche als Kosmetikum zum Auftragen auf die menschliche Haut, bevorzugt die Hand.

15. Verwendung einer Emulsion nach einem der vorstehenden Ansprüche als Handcreme, Fußcreme, Mousseapplikation mit/ohne Treibgas oder Pflegelotion.

16. Verwendung einer Emulsion nach einem der vorstehenden Ansprüche zur Verbesserung und/oder Steigerung der Hautfeuchte (KAP) und/oder Hautglätte (MTG).

17. Verfahren zur Verbesserung und/oder Steigerung der Hautfeuchte und/oder Hautglätte, **dadurch gekennzeichnet, dass** eine Emulsion nach einem der Ansprüche 1 bis 13 auf die Haut, insbesondere die Hand, aufgetragen wird.

18. Emulsion nach einem der Ansprüche 1 bis 13 zur Herstellung eines Mittels zur Pflege, Linderung spröder, rissiger und/oder entzündeter Haut, bevorzugt der Hand.

## Claims

1. O/W emulsion comprising fatty alcohol and/or fatty alcohol mixtures, at least one moisturizing agent, where the fraction of moisturizing agent is at least 25% by weight, based on the total mass of the emulsion, at least one glycerol ester of a fatty acid and at least one phosphate emulsifier.

2. Emulsion according to Claim 1, **characterized in that** glycerol is selected as moisturizing agent.

3. Emulsion according to one of the preceding claims, **characterized in that** the fatty alcohols or fatty alcohol mixtures have a melting point in the range from about 32°C to 56°C.

4. Emulsion according to one of the preceding claims, **characterized in that** the fraction of fatty acid glycerol esters is at least about 0.1% by weight, preferably at least about 0.5% by weight, preferably at most about 5% by weight, in particular at most 3% by weight, based on the total mass of the emulsion, and/or the fraction of fatty alcohol or fatty alcohol mixtures is selected to be at least about 2% by weight, preferably at most up to 10% by weight, based on the total mass of the emulsion.

5. Emulsion according to one of the preceding claims, **characterized in that** the fatty acid glycerol ester or esters have a carbon chain length of less than 22.

6. Emulsion according to one of the preceding claims, **characterized in that** glyceryl laurate is selected as fatty acid glycerol ester.

7. Emulsion according to one of the preceding claims, **characterized in that** the fraction of phosphate emulsifiers is selected to be at least about 0.1% by weight, preferably at least 0.5% by weight, preferably at most 5% by weight, in particular at most 3% by weight, based on the total mass of the emulsion.

8. Emulsion according to one of the preceding claims, **characterized in that** trilaureth-4 phosphate and/or triceteareth-4 phosphate are selected as phosphate emulsifiers.

9. Emulsion according to one of the preceding claims, **characterized in that** additional skincare, wound-healing, anti-inflammatory and/or pain-relieving substances are present.

10. Emulsion according to Claim 9, **characterized in that** panthenol is present, preferably a fraction of from 0.1 to 5% by weight, in particular 0.5 to 2% by weight, based on the total mass of the emulsion.

11. Emulsion according to one of the preceding Claims 9 to 10, **characterized in that** calendula oil, preferably about 0.01 to 5% by weight, in particular 0.1 to 4% by weight, based on the total mass of the emulsion, is present.

12. Emulsion according to one of the preceding claims, **characterized in that** glycerol, panthenol and calendula oil are present.

13. Emulsion according to one of the preceding claims in the form of a cream, a lotion, a gel, an ointment, a tincture, a milk, a balm, a plaster impregnated with the emulsion, a wipe impregnated with the emulsion, a textile impregnated with the emulsion, a pad impregnated with the emulsion, a spray, an aerosol, a roll-on, a stick, a soft solid, a powder or a powder spray.

14. Use of an emulsion according to one of the preceding claims, as cosmetic for application to the human skin, preferably the hand.

15. Use of an emulsion according to one of the preceding claims as hand cream, foot cream, mousse application with/without propellant gas or care lotion.

16. Use of an emulsion according to one of the preceding claims for improving and/or increasing skin moisture (KAP) and/or skin smoothness (MTG).

17. Method of improving and/or increasing skin moisture and/or skin smoothness, **characterized in that** an emulsion according to one of Claims 1 to 13 is applied to the skin, in particular the hand.

18. Emulsion according to one of Claims 1 to 13 for producing a composition for the care, alleviation of rough, chapped and/or inflamed skin, preferably of the hand.

## Revendications

1. Émulsion H/E comprenant un alcool gras et/ou des mélanges d'alcools gras, au moins un agent hydratant, la teneur en agent hydratant étant d'au moins 25 % en poids, par rapport à la masse totale de l'émulsion, au moins un ester de glycérol avec un acide gras et au moins un émulsifiant phosphate.

2. Émulsion selon la revendication 1, **caractérisée en ce qu'**on choisit comme agent hydratant le glycérol.

3. Émulsion selon l'une quelconque des revendications précédentes, **caractérisée en ce que** les alcools gras ou mélanges d'alcools gras ont un point de fusion dans la plage d'environ 32 °C à 56 °C.

4. Émulsion selon l'une quelconque des revendications précédentes, **caractérisée en ce que** la teneur en ester d'acide gras et de glycérol est d'au moins environ 0,1 % en poids, de préférence d'au moins environ 0,5 % en poids, de préférence d'au maximum environ 5 % en poids, en particulier d'au maximum 3 % en poids, par rapport à la masse totale de l'émulsion, et/ou la teneur en alcool gras ou mélanges d'alcools gras est choisie au moins d'environ 2 % en poids, de préférence au maximum jusqu'à 10 % en poids, par rapport à la masse totale de l'émulsion.

5. Émulsion selon l'une quelconque des revendications précédentes, **caractérisée en ce que** l'ester ou les esters d'acide gras et de glycérol ont une longueur de chaîne carbonée de moins de 22.

6. Émulsion selon l'une quelconque des revendications précédentes, **caractérisée en ce qu'**on choisit comme ester d'acide gras et de glycérol le laurate de glycéryle.

7. Émulsion selon l'une quelconque des revendications précédentes, **caractérisée en ce que** la teneur en émulsifiants phosphate est choisie d'au moins environ 0,1 % en poids, de préférence d'au moins 0,5 % en poids, de préférence d'au maximum 5 % en poids, en particulier d'au maximum 3 % en poids, par rapport à la masse totale de l'émulsion.

8. Émulsion selon l'une quelconque des revendications précédentes, **caractérisée en ce qu'**on choisit comme émulsifiants phosphate le phosphate de trilaureth-4 ou le phosphate de tricétéareth-4.

9. Émulsion selon l'une quelconque des revendications précédentes, **caractérisée en ce que** des substances supplémentaires pour le soin des mains, cicatrisantes, anti-inflammatoires et/ou analgésiques sont contenues.

10. Émulsion selon la revendication 9, **caractérisée en ce que** du panthénol est contenu, de préférence en une proportion de 0,1 à 5 % en poids, en particulier de 0,5 à 2 % en poids, par rapport à la masse totale de l'émulsion.

11. Émulsion selon l'une quelconque des revendications précédentes, **caractérisée en ce que** de l'huile de calendula est contenue, de préférence en une proportion d'environ 0,01 à 5 % en poids, en particulier de 0,1 à 4 % en poids, par rapport à la masse totale de l'émulsion.

12. Émulsion selon l'une quelconque des revendications précédentes, **caractérisée en ce que** du glycérol, du panthénol et de l'huile de calendula sont contenus.

13. Émulsion selon l'une quelconque des revendications précédentes, sous forme d'une crème, d'une lotion, d'un gel, d'une pommade, d'une teinture, d'un lait, d'un baume, d'un pansement adhésif imprégné de l'émulsion, d'une lingette imprégnée de l'émulsion, d'un textile imprégné de l'émulsion, d'un coussin imprégné de l'émulsion, d'une composition à pulvériser en aérosol, d'un aérosol, d'une composition à appliquer à l'applicateur à bille, d'un bâton, d'un Soft Solid, d'une poudre ou d'une composition à pulvériser en poudre.

14. Utilisation d'une émulsion selon l'une quelconque des revendications précédentes, en tant que cosmétique destiné à l'application sur la peau humaine, de préférence la main.

15. Utilisation d'une émulsion selon l'une quelconque des revendications précédentes, en tant que crème pour les mains, crème pour les pieds, application en mousse avec/sans gaz propulseur ou lotion de soin.

16. Utilisation d'une émulsion selon l'une quelconque des revendications précédentes, pour l'amélioration et/ou l'augmentation de l'humidité de la peau (KAP) et/ou du lissé de la peau (MTG)

17. Procédé pour l'amélioration et/ou l'augmentation de l'humidité de la peau et/ou du lissé de la peau, **caractérisé en ce qu'**on applique sur la peau, en particulier la main, une émulsion selon l'une quelconque des revendications 1 à 13.

18. Émulsion selon l'une quelconque des revendications 1 à 13, pour la fabrication d'un produit pour le soin, l'apaisement de la peau rêche, gercée et/ou enflammée, de préférence de la main.
